# EUROPEAN PATENT APPLICATION

(11) **EP 4 649 996 A1**
(43) Date of publication of application: **19.11.2025**
(21) Application number: 24744384.9
(22) Date of filing: 19.01.2024
(51) Int. Cl.: A61N 5/10

(54) **RADIATION THERAPY SYSTEM AND POSITIONING METHOD THEREFOR**

(30) Priority: 19.01.2023 CN 202310074088; 18.01.2024 CN 202410075343
(71) Applicant: Neuboron Therapy System Ltd., Xiamen, Fujian 361026 (CN)
(72) Inventor: SHU, Diyun, Nanjing, Jiangsu 211112 (CN); LIU, Yuanhao, Nanjing, Jiangsu 211112 (CN); GONG, Qiuping, Nanjing, Jiangsu 211112 (CN); LIU, Xingyan, Nanjing, Jiangsu 211112 (CN)
(74) Representative: Gunzelmann, Rainer
(86) International application number: PCT/CN2024/073154
(87) International publication number: WO 2024/153214

(57) **Abstract**

A radiation therapy system, especially, a neutron capture therapy system, and a positioning method thereof are provided. The positioning method includes: determining a target position of a to-be-irradiated body based on a treatment plan; moving the to-be-irradiated body to a positioning position according to the target position; obtaining second image data of the to-be-irradiated body and a reference object; determining first position information based on the treatment plan, and determining second position information based on the second image data; calculating a deviation value between the positioning position and the target position based on the first position information and the second position information; and adjusting a position of the to-be-irradiated body based on the deviation value. A positioning verification method and a correction method of a radiation therapy system are further included, so that a positioning deviation can be verified and corrected, thereby ensuring safety and accuracy of radiation therapy.

## Description

### TECHNICAL FIELD

This application relates to the field of radiation medical technologies, and in particular, to a radiation therapy system and a positioning method thereof.

### BACKGROUND

With the development of atomic science, radiation therapy such as Cobalt 60, linear accelerator, and electron beam has become one of the main means of cancer therapy. However, conventional photon or electron therapy is limited by physical conditions of the radiation. When neoplastic cells are killed, a large quantity of normal tissues in a beam path are also damaged. In addition, due to different degrees of sensitivity of neoplastic cells to radiation, the conventional radiation therapy usually has poor effects on radiation-resistant malignant neoplasms (such as Glioblastoma Multiforme and Melanoma).

To reduce radiation damage to normal tissues around the neoplasm, a concept of targeted therapy in chemotherapy is applied to the radiation therapy. Radiation sources, for example, proton therapy, heavy particle therapy, and neutron capture therapy, having a high relative biological effectiveness (RBE) are also actively developed currently for highly-radiation-resistant neoplastic cells. The neutron capture therapy combines the foregoing two concepts. For example, boron neutron capture therapy (BNCT for short), by means of specific aggregation of boron-containing medicine at the neoplastic cell in cooperation with precise neutron beam control, provides a selection of cancer therapy better than the conventional radiation therapy.

In a BNCT therapy process, after a to-be-irradiated body is moved, by using a positioning apparatus such as a therapy bed or a therapy chair, to an actual therapy or simulated positioning position according to a target position in a treatment planning solution developed by a treatment planning system (TPS), the actual therapy or simulated positioning position may still deviate from the preset target position in the treatment plan due to factors such as a fixed deviation, a human mistake, and a device failure. Once therapy is incorrectly started, it easily causes an inaccurate dose granted to the to-be-irradiated body, affecting a therapy effect or causing excessive irradiation, and causing adverse effects.

Therefore, a technical means is required to verify and correct positioning accuracy, and the technical means should have features such as short operating time, high accuracy, and simple operation.

### SUMMARY

In view of this, for the foregoing problem, it is necessary to provide an accurate radiation therapy system, and in particular, a neutron capture therapy system and a positioning method thereof.

According to a first aspect, this application provides a radiation therapy system, including: an irradiation module, configured to: generate a therapy beam, and irradiate the therapy beam to a to-be-irradiated body; an image module, configured to obtain image data of the to-be-irradiated body; a treatment planning module, configured to generate a treatment plan based on the image data; a positioning module, configured to move the to-be-irradiated body to a positioning position according to a target position obtained by using the treatment plan, where the image data includes at least second image data of the to-be-irradiated body and a reference object at the positioning position; and a processing module, configured to: determine first position information based on the treatment plan, determine second position information based on the second image data, and calculate a deviation value between the positioning position and the target position based on the first position information and the second position information.

In an embodiment, a control module is included, configured to control the positioning module to adjust a position of the to-be-irradiated body based on the deviation value.

In an embodiment, when the control module controls the positioning module to adjust the position of the to-be-irradiated body based on the deviation value, if the deviation value is less than or equal to a preset threshold or the deviation value can be adjusted to be less than or equal to a preset threshold, a positioning verification succeeds; and if the deviation value is always greater than the threshold, the positioning verification fails, and the treatment plan needs to be re-adjusted.

In an embodiment, a marker is included, and the marker is disposed or selected on the to-be-irradiated body.

In an embodiment, there are at least three markers, and any three of the markers are not on a straight line.

In an embodiment, there are at least four markers, and at least one of the markers is not in the same plane as other markers.

In an embodiment, the image module includes a first image module, where the first image module is configured to obtain first image data of the to-be-irradiated body and the marker, and the first image data is used for determining first position information in coordination with the treatment plan.

In an embodiment, the first image module obtains image information of the to-be-irradiated body and/or the reference object and the marker by using a transmissive electromagnetic wave signal, to form the first image data.

In an embodiment, the image module further includes a second image module, the second image module is different from the first image module, the second image module includes at least one sensor and at least one light source, the light source is configured to at least emit light in a first spectral range, the first spectral range is near infrared light, and the second image module obtains image information on surfaces of the to-be-irradiated body, the reference object, and the marker by using information reflected from the light source to the sensor, and forms the second image data.

In an embodiment, the second image module is a mobile image device.

In an embodiment, the second image module is a handheld scanning device, including at least two sensors and at least two light sources. A spectral range of at least one light source is non-visible light, and a spectral range of the other light source is visible light.

In an embodiment, the image module obtains first image data of the to-be-irradiated body and the marker; and the treatment planning module includes a preset virtual reference object model, and the treatment plan determines the target position based on the first image data and the virtual reference object model.

In an embodiment, the first position information includes first relative position information between the to-be-irradiated body and the virtual reference object model, and first marker position information describing the marker; and the second position information includes second relative position information between the to-be-irradiated body and the reference object, and second marker position information describing the marker.

In an embodiment, the deviation value includes a first deviation value and a second deviation value, the processing module obtains the first deviation value through calculation based on the first relative position information and the second relative position information, the processing module obtains the second deviation value through calculation based on the first marker position information and the second marker position information, and the control module controls the positioning module to adjust the position of the to-be-irradiated body based on the first deviation value and/or the second deviation value.

In an embodiment, the virtual reference object model is a virtual beam exit image three-dimensional model preset by the treatment planning module, and the reference object is the beam exit.

In an embodiment, the to-be-irradiated body is fixed at a corresponding position on the positioning module by using a position limiting member.

In an embodiment, the marker includes directly marking the to-be-irradiated body, or marking the position limiting member.

According to a second aspect, this application provides a positioning method of a radiation therapy system. The positioning method includes: determining a target position of a to-be-irradiated body based on a treatment plan; moving the to-be-irradiated body to a positioning position according to the target position; obtaining second image data of the to-be-irradiated body and a reference object; determining first position information based on the treatment plan, and determining second position information based on the second image data; calculating a deviation value between the positioning position and the target position based on the first position information and the second position information; and adjusting a position of the to-be-irradiated body based on the deviation value.

In an embodiment, the adjusting a position of the to-be-irradiated body based on the deviation value includes: when adjusting the position of the to-be-irradiated body based on the deviation value, if the deviation value is less than or equal to a preset threshold or the deviation value can be adjusted to be less than or equal to a preset threshold, a positioning verification succeeds; and if the deviation value is always greater than the threshold, the positioning verification fails, and the treatment plan needs to be re-adjusted.

In an embodiment, the radiation therapy system includes a first image module, and the determining a target position of a to-be-irradiated body based on a treatment plan includes: disposing or selecting a marker on the to-be-irradiated body; and obtaining first image data of the to-be-irradiated body and the marker by using the first image module; and the treatment plan includes a preset virtual reference object model, and the treatment plan determines the target position based on the first image data and the virtual reference object model.

In an embodiment, the first position information includes first relative position information between the to-be-irradiated body and the virtual reference object model, and first marker position information describing the marker; and the second position information includes second relative position information between the to-be-irradiated body and the reference object, and second marker position information describing the marker.

In an embodiment, the deviation value includes a first deviation value and a second deviation value; the first deviation value is obtained through calculation based on the first relative position information and the second relative position information, the second deviation value is obtained through calculation based on the first marker position information and the second marker position information, and the deviation value may include deviation values of positions and/or angles.

In an embodiment, the threshold includes a first threshold and a second threshold, and the adjusting a position of the to-be-irradiated body based on the deviation value includes: when the first deviation value is less than or equal to the first threshold, and the second deviation value is less than or equal to the second threshold, positioning verification is determined to be completed; and when the first deviation value is greater than the first threshold, and/or the second deviation value is greater than the second threshold, the control module controls, based on the first deviation value and/or the second deviation value, the positioning module to adjust the position of the to-be-irradiated body, until the first deviation value is less than or equal to the first threshold and the second deviation value is less than or equal to the second threshold.

In an embodiment, the threshold includes a first threshold and a second threshold, and the adjusting a position of the to-be-irradiated body based on the deviation value includes: when the first deviation value is greater than the first threshold, the control module controls, based on the first deviation value, the positioning module to adjust the position of the to-be-irradiated body, until the first deviation value is less than or equal to the first threshold; and when the second deviation value is greater than the second threshold, the control module controls, based on the second deviation value, the positioning module to adjust the position of the to-be-irradiated body, until the second deviation value is less than or equal to the second threshold.

In an embodiment, the threshold includes a first threshold and a second threshold, and the adjusting a position of the to-be-irradiated body based on the deviation value includes: when the first deviation value is less than or equal to the first threshold, and the second deviation value is greater than the second threshold, the control module controls, based on the second deviation value, the positioning module to adjust the position of the to-be-irradiated body, until the second deviation value is less than or equal to the second threshold.

In an embodiment, before the calculating a deviation value between the positioning position and the target position based on the first position information and the second position information, the method further includes: registering the image three-dimensional model obtained by re-establishing the first image data and the second image data, so that the model obtained by re-establishing the reference object coincides with the virtual reference object model.

In an embodiment, before the determining a target position of a to-be-irradiated body based on a treatment plan, the method further includes: establishing a first image three-dimensional model of the to-be-irradiated body based on the first image data; and establishing a second image three-dimensional model of the to-be-irradiated body based on the second image data.

In an embodiment, the adjusting a position of the to-be-irradiated body based on the deviation value includes: establishing a first three-dimensional parent coordinate system by using a central point of a reference object model as an origin; establishing a second three-dimensional parent coordinate system having a dimension the same as that of the first three-dimensional parent coordinate system by using a central point of the virtual reference object model as an origin; establishing a first three-dimensional child coordinate system based on information about the marker in the first image data, and establishing a second three-dimensional child coordinate system having an origin and a dimension the same as those of the first three-dimensional child coordinate system based on information about the marker in the second image data; when the first three-dimensional parent coordinate system and the second three-dimensional parent coordinate system are completely registered, integrating the first three-dimensional child coordinate system with the first three-dimensional parent coordinate system, and integrating the second three-dimensional child coordinate system with the second three-dimensional parent coordinate system; and calculating a deviation value between the first three-dimensional child coordinate system and the second three-dimensional child coordinate system, and verify and adjust the position of the to-be-irradiated body based on the deviation value.

In an embodiment, the first image data and/or the second image data is obtained by using a mobile image device or a CT device.

According to a third aspect, this application provides a neutron capture therapy system, including:
a neutron generation module, where the neutron generation module includes an accelerator and a target, and a charged particle beam generated by the accelerator through acceleration reacts with the target to generate a neutron beam;
a beam shaper module, where the beam shaper module includes a reflector, a moderator, a thermal neutron absorber, a radiation shield, and a beam channel, the moderator decelerates neutrons generated from the target to an epithermal neutron energy area, the reflector surrounds the moderator and guides deviating neutrons back to the moderator, to improve epithermal neutron beam intensity, the thermal neutron absorber is configured to absorb thermal neutrons to avoid an excessive dose caused to superficial normal tissues during therapy, and the radiation shield is disposed around the beam channel to shield leaking neutrons and photons to reduce a dose on normal tissues in a non-irradiated area;
an image module, configured to obtain image data of a to-be-irradiated body;
a treatment planning module, configured to generate a treatment plan based on the image data;
a positioning module, configured to move the to-be-irradiated body to a positioning position according to a target position obtained by using the treatment plan, where the image data includes at least second image data of the to-be-irradiated body and a reference object at the positioning position; and
a processing module, configured to: determine first position information based on the treatment plan, determine second position information based on the second image data, and calculate a deviation value between the positioning position and the target position based on the first position information and the second position information.

According to the radiation therapy system, in particular, the neutron capture therapy system, and the positioning method thereof involved in this application, positioning accuracy of a to-be-irradiated body can be quickly and accurately verified, and correction data can be provided based on image data when a positioning deviation occurs, to ensure that the to-be-irradiated body reaches a planned therapy position to a maximum extent. If it is found in an actual operation process that it is difficult to position the to-be-irradiated body to the planned therapy position, the dose may also be corrected after the therapy based on positioning deviation data. It can be learned from the foregoing features of this application that a possibility of occurrence of a positioning deviation can be reduced, and in particular, an accurate BNCT therapy dose can be ensured to be given, to ensure a BNCT therapy effect, and avoid excessive irradiation on normal tissues, thereby further improving safety and accuracy of the BNCT technology.

### BRIEF DESCRIPTION OF THE DRAWINGS

FIG. 1 is a schematic diagram of modules in a radiation therapy system according to this application;
FIG. 2 is a schematic structural diagram of a radiation therapy system according to this application;
FIG. 3 is a schematic diagram of a to-be-irradiated body and a marker according to an embodiment of this application;
FIG. 4 is a schematic flowchart of positioning method according to an embodiment of this application;
FIG. 5a is a schematic diagram of a first image three-dimensional model and a virtual reference object model according to an embodiment of this application;
FIG. 5b is a schematic diagram of a positioning position according to an embodiment of this application;
FIG. 5c is a schematic diagram of a positioning position according to an embodiment of this application;
FIG. 6 is a schematic flowchart of S800 according to an embodiment of this application; and
FIG. 7 is a schematic flowchart of another embodiment according to this application.

### Descriptions of reference numerals:

100: Radiation therapy system; 10: irradiation module; 20: treatment planning module; 30: control module; 40: processing module; 50: image module; 200: to-be-irradiated body; 101: therapy room; 102: beam generation room;
11: neutron generation apparatus; 12: beam shaper; 13: beam exit; 14: positioning module;
121: reflector; 122: moderator; 123: thermal neutron absorber; 124: radiation shield; 125: beam channel;
130: virtual reference object model; 130': reference object; 131: simulated beam exit; 131': beam exit; 132, 132': exit central line; 133, 133': exit central point; 201: position limiting member; 300: marker.

### DETAILED DESCRIPTION

To make the objectives, technical solutions, and advantages of this application clearer, the following further describes this application in detail with reference to the accompanying drawings and the embodiments. The specific embodiments described herein are only used to explain this application, and are not intended to limit this application.

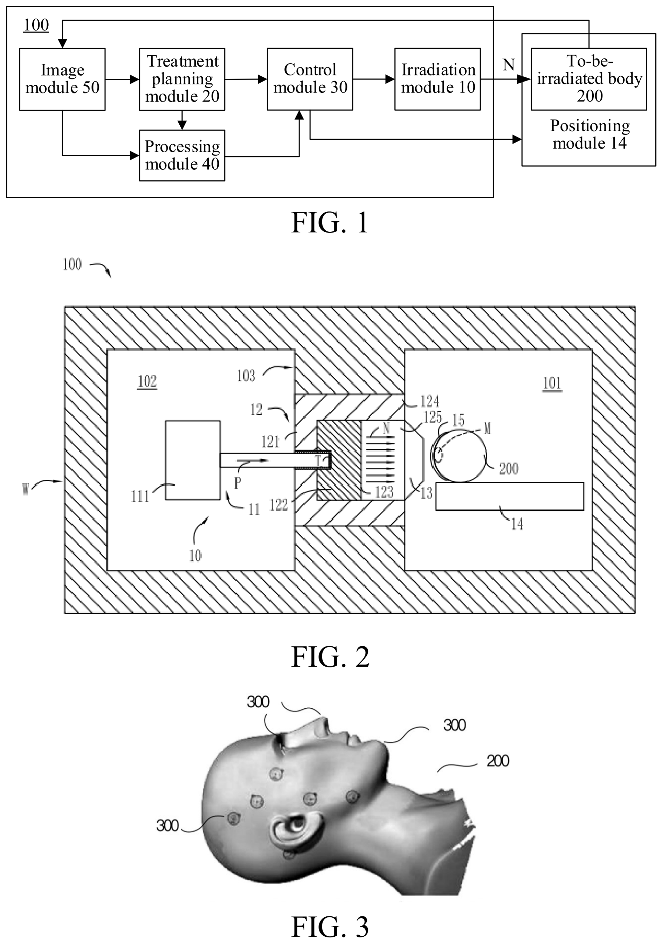

Referring to FIG. 1, FIG. 1 is a schematic diagram of a radiation therapy system 100 performing irradiation therapy on a to-be-irradiated body 200. The radiation therapy system includes an irradiation module 10, a treatment planning module 20, a control module 30, a processing module 40, an image module 50, and a positioning module 14. The radiation therapy system 100 is configured to perform radiation irradiation on the to-be-irradiated body 200. The radiation therapy system 100 in this embodiment is preferably a boron neutron capture therapy system, and the irradiation module 10 is set to be a neutron beam irradiation module in a matching manner. In another embodiment, the irradiation module 10 may further use protons, heavy ions, or the like as a radiation beam for disease therapy.

The image module 50 is configured to recognize the to-be-irradiated body 200 and generate image data, and the treatment planning module 20 generates a treatment plan based on the image data obtained by the image module 50. The processing module 50 determines first position information based on the treatment planning module 20 and the first image data, obtains and determines second position information based on second image data, calculates a deviation value between a positioning position and a target position based on the first position information and the second position information, and instructs the positioning module 14 or a user to adjust a position of the to-be-irradiated body 200. Further, the image module 50 may be a mobile image device, a handheld image device, or the like. In another embodiment, the image module 50 includes a first image module and a second image module. Preferably, the first image module is different from the second image module. Further, the first image module and the second image module may be disposed in different preparation rooms or in a same preparation room in a movable or fixed manner, to respectively obtain the first image data and the second image data.

In another embodiment, a movable handheld image device is used as the second image module. This is more convenient for one device to be reused in a plurality of different spaces, and does not need installation or position adjustment. Therefore, it is more advantageous in convenience compared with a fixed image device. This is beneficial to miniaturization and compactness of deployment inside a BNCT facility, improves space utilization, reduces a occupied area and building construction costs, and avoids a measurement error of the second image module caused by radiation in a high-radiation environment for long time.

With reference to FIG. 2, the irradiation module 10 is configured to generate a therapy neutron beam N and irradiate the therapy neutron beam N to a to-be-irradiated position of the to-be-irradiated body 200. The treatment planning module 20 performs dose simulation calculation based on medical image data of the to-be-irradiated position and generates an initial treatment plan. The treatment plan determines an irradiation dose on the to-be-irradiated position, a position of the to-be-irradiated position relative to the irradiation module 10 during irradiation therapy, corresponding irradiation time, and the like. Usually, after the to-be-irradiated body 200 is positioned to a planned target position according to the position determined by the treatment plan, the therapy may be started. The control module 30 invokes a treatment plan suitable for the current to-be-irradiated body 200 from the treatment planning module 20, and controls, according to the treatment plan, the irradiation module 10 to perform irradiation. The control module 30 may further receive other data information, such as data of the irradiation module 10, data of the to-be-irradiated body 200, and data of the positioning module 14. As shown in FIG. 2, the irradiation module 10 includes a neutron generation apparatus 11, a beam shaper 12, a beam exit 13, and a positioning module 14. The neutron generation apparatus 11 includes an accelerator 111, a target T, and the like. The accelerator 111 accelerates charged particles (such as protons and deuterons), to generate a charged particle ray P such as a proton ray. The charged particle ray P is irradiated on the target T and reacts with the target T to generate a neutron ray (neutron beam) N. The target T is preferably a metal target. A suitable nuclear reaction is selected based on features such as a required neutron production rate and energy, energy and a current of accelerated charged particles that can be provided, and physicochemical properties of the metal target. The nuclear reaction that is usually discussed includes ⁷Li(p, n)⁷Be and ⁹Be(p, n)⁹B, where both the reactions are endothermic reactions. Energy thresholds of the two nuclear reactions are respectively 1.881 MeV and 2.055 MeV. Because an ideal neutron source for the boron neutron capture therapy is an epithermal neutron with an energy level of keV, theoretically, if a lithium metal target is bombarded by a proton with an energy only slightly higher than the threshold, a low-energy neutron may be generated, and the low-energy neutron can be used in clinic without much deceleration processing. However, two targets: lithium metal (Li) and beryllium metal (Be) has low action cross section with a proton at threshold energy. To generate a sufficiently large neutron flux, generally, a proton with high energy is selected for triggering the nuclear reaction. An ideal target should have features such as having a high neutron production rate, generating a neuron with energy distribution close to an epithermal neutron energy area, not generating excessively much penetrating radiation, being safe, cheap, and easy to operate, and hightemperature resistance. However, actually, a nuclear reaction satisfying all the requirements cannot be found. A target made of lithium metal is preferably used in the embodiments of this application. However, as well-known by a person skilled in the art, the material of the target T may alternatively be a metal material other than lithium or berylum, for example, may be tantalum (Ta) or tungsten (W). The target T may be in a circular plate shape, or may be in another solid shape, or may be a liquid (liquid metal). The accelerator 111 may be a linear accelerator, a cyclotron, a synchrotron, or a synchrocyclotron. Further, the neutron generation apparatus 11 may be a nuclear reactor rather than an accelerator and a target. Regardless of whether the neutron source of the boron neutron capture therapy is from a nuclear reactor or from a nuclear reaction between a charged particle in an accelerator and a target, a generated radiation field is actually a hybrid radiation field, in other words, a beam includes low-energy to high-energy neutrons and photons.

The neutron beam N generated by the neutron generation apparatus 11 is irradiated to the to-be-irradiated body 200 on the positioning module 14 sequentially through the beam shaper 12 and the beam exit 13. The beam shaper 12 can adjust beam quality of the neutron beam N generated by the neutron generation apparatus 11. The beam exit 13 may be a structure of a collimator, and is configured to converge the neutron beam N, so that the neutron beam N has high targetability in the therapy process. It may be understood that, a collimator may alternatively be not included in this application, and after exiting from the beam shaper 12, the beam is directly irradiated to the to-be-irradiated body 200 on the positioning module 14 through the beam exit. The positioning module 14 is configured to: move and accommodate the to-be-irradiated body 200 to position the to-be-irradiated body 200, and move the to-be-irradiated body to a positioning position according to the target position obtained by using the treatment plan. In an embodiment, the positioning module 14 adjusts and positions the to-be-irradiated body 200 according to the target position, so that the to-be-irradiated body 200 can receive the neutron beam N stably at an appropriate position, and the positioning module can automatically or operatively adjust the position of the to-be-irradiated body 200. Further, the positioning module 14 may include a therapy bed, a therapy chair, or the like. The positioning module 14 may further include an adjustment mechanism connecting the therapy bed, the therapy chair, or the like, for example, a mechanical arm configured to automatically adjust a position of the positioning module 14, which is not shown in the figure.

The beam shaper 12 further includes a reflector 121, a moderator 122, a thermal neutron absorber 123, a radiation shield 124, and a beam channel 125. Because the neutron generated by the neutron generation apparatus 11 has a very wide energy spectrum, other than epithermal neurons satisfying a therapy requirement, quantities of other types of neutrons and photons need to be reduced as much as possible, to avoid damage to operating personnel or the to-be-irradiated body. Therefore, a neutron exiting from the neutron irradiation apparatus 10 needs to pass through the moderator 22 to decelerate neutrons generated by the target to an epithermal neutron energy area, in other words, adjust fast neutron energy (> 10 keV) of the neutron to the epithermal neutron energy area (0.5 eV to 10 keV), and reduce thermal neutrons (< 0.5 eV) as much as possible. The moderator 22 is made of a material having a large reaction cross section with the fast neutron and a small reaction cross section with the epithermal neutron. In a preferred embodiment, the moderator 22 is made of at least one of D₂O, AlF₃, Fluental^{™}, CaF₂, Li₂CO₃, MgF₂, and Al₂O₃. The reflector 121 surrounds the moderator 122, and reflects, back to the moderator and back to the neutron beam N, neutrons diffusing and deviating after passing through the moderator 122, to improve neutron utilization, and the reflector is made of a material having a strong neutron reflecting capability. In a preferred embodiment, the reflector 121 is made of at least one of Pb or Ni. There is a thermal neutron absorber 123 at a rear portion of the moderator 122, and the thermal neutron absorber 123 is made of a material having a large reaction cross section with the thermal neutron. In a preferred embodiment, the thermal neutron absorber is made of Li-6. The thermal neutron absorber 123 is configured to absorb thermal neutrons passing through the moderator 122, to reduce a quantity of thermal neutrons in the neutron beam N, and prevent causing an excessive dose for superficial normal tissues. It may be understood that, the thermal neutron absorber may alternatively be integrated with the moderator, and a material of the moderator includes Li-6. The radiation shield 124 is disposed around the beam exit, and is configured to shield neutrons and photons leaking from a part other than the beam channel 125, to reduce a dose of normal tissues in a non-irradiated area, and a material of the radiation shield 124 includes at least one of a photon shielding material and a neutron shielding material. In a preferred embodiment, the material of the radiation shield 2214 includes a photon shielding material: lead (Pb), and a neutron shielding material: polyethylene (PE). The beam exit 13 is disposed at a rear portion of the beam channel 125. An epithermal neutron beam exiting from the beam exit 13 is irradiated to the to-be-irradiated body 200, and after passing through superficial normal tissues, the epithermal neutron beam is decelerated to thermal neutrons, and reaches neoplastic cells in an affected part M. It may be understood that the beam shaper 20 may alternatively have another construction, provided that an epithermal neutron beam required for the therapy can be obtained. For ease of description, when the beam exit 13 is provided, the beam exit 13 is disposed downstream of the beam channel 125. In this embodiment, a radiation shielding apparatus 15 may further be disposed between the to-be-irradiated body 200 and the beam exit 13, to shield radiation from a beam exiting from the beam exit 13 to normal tissues of the to-be-irradiated body. It may be understood that, the radiation shielding apparatus 15 may alternatively not be disposed. For the boron neutron capture therapy of deep neoplasm, a larger quantity of radiant rays other than epithermal neurons causes a larger proportion of non-selective dose deposition of normal tissues. Therefore, the radiation that causes unnecessary dose should be reduced as much as possible. In addition, for the normal tissues of the to-be-irradiated body, excessively many radiant rays which also causes unnecessary dose deposition should be avoided.

After the to-be-irradiated body 200 takes or is injected with boron (B-10)-containing medicine, the boron-containing medicine selectively accumulates in neoplastic cells in an affected part M. Then, because the boron (B-10)-containing medicine features in high capture cross section for thermal neurons, two heavy charged particles: ⁴He and ⁷Li are generated through ¹⁰B(n, α)⁷Li neutron capture and nuclear fission reaction. Average energy of the two heavy charged particles is approximately 2.33 MeV, and the two particles have features of high linear energy transfer (LET) and short ranges, where linear energy transfer and a range of a particle α are respectively 150 keV/µm and 8 µm, linear energy transfer and a range of a heavy charged particle ⁷Li are respectively 175 keV/µm and 5 µm, and a total range of the two particles is approximately equivalent to a size of a cell. Therefore, radiation damage caused to a living body can be limited to a cell level, and this can achieve an objective of locally killing neoplastic cells without causing too much damage to normal tissues.

Referring to FIG. 2, the radiation therapy system 100 is integrally accommodated in a building constructed by concrete. Specifically, the radiation therapy system 100 further includes a therapy room 101 and a beam generation room 102. The to-be-irradiated body 200 on the positioning module 14 receives a therapy of neutron beam N irradiation in the therapy room 101. The beam generation room 102 at least partially accommodates the accelerator 111. The beam shaper 12 is at least partially accommodated in a partition wall 103 between the therapy room 101 and the beam generation room 102. It may be understood that the partition wall 103 may completely separate the therapy room 101 and the beam generation room 102. Alternatively, may partially separate the therapy room 101 and the beam generation room 102, and the therapy room 101 communicates with the beam generation room 102. There may be one or more targets T. The charged particle ray P optionally interacts with one or more targets T, or interacts with a plurality of targets T at the same time, to generate one or more therapy neutron beams N. In correspondence to the quantity of the targets T, there may also be one or more beam shapers 12, beam exits 13, and positioning modules 14. A plurality of positioning modules may be disposed in a same therapy room, or an independent therapy room may be disposed for each positioning module. The therapy room 101 and the beam generation room 102 are spaces surrounded by a concrete wall W (including the partition wall 103). The concrete structure can shield neutrons and other radiant rays leaked in an operating process of the radiation therapy system 100.

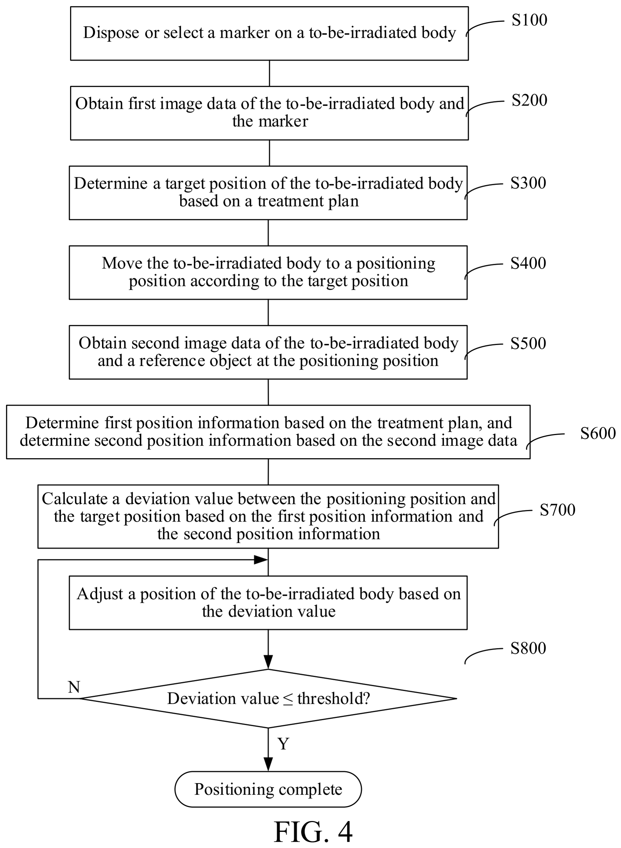

Before performing the radiation therapy, whether the to-be-irradiated body is positioned at an appropriate therapy position needs to be determined. Specifically, whether the positioning position of the to-be-irradiated body relative to the beam exit 13 after being positioned is suitable for the radiation therapy needs to be verified, and whether the positioning position reaches the target position preset in the treatment plan needs to be verified. When the to-be-irradiated body performs the irradiation therapy at an appropriate position, the radiant rays can kill neoplastic cells in the to-be-irradiated body to the maximum extent, and damage of the radiant rays to surrounding normal tissues is reduced as much as possible. FIG. 4 is a schematic flowchart of a positioning method based on a radiation therapy system according to an embodiment of this application, specifically including the following method:
S100: Dispose or select a marker 300 on a to-be-irradiated body 200.

As shown in FIG. 3, a marker 300 is disposed or selected on the to-be-irradiated body 200, and is used as a reference when subsequently forming image data and being positioned, to calibrate and align a position of the to-be-irradiated body 200.

The marker 300 may be disposed on the to-be-irradiated body 200 by means of biological or chemical attachment, or may be disposed on the to-be-irradiated body 200 by means of physical attachment. Preferably, the marker 300 is disposed on the to-be-irradiated body 200 non-invasively, for example, is visibly marked on a surface of the to-be-irradiated body. This helps the marker 300 to be displayed in a subsequent image, and also helps the marker to be visibly aligned during subsequent position switching and positioning. In this embodiment, the marker 300 is preferably made of a metal material, for example, made of a material such as lead or surgical steel, or may be marked by using plastic, a label, a cast, or drawn by using a pencil, a marker pen, or a paint. In another embodiment, the marker 300 may alternatively be a marking feature of the to-be-irradiated body 200. For example, as shown in FIG. 3, when the to-be-irradiated body is a patient, the marker may be selected to be a prominent visual characteristic point such as a head contour line, a nose tip, an eyeball, or a mandible of a human body. The marker 300 may be selected by the processing module 40, or may be externally inputted by using the radiation therapy system 100. This is not limited herein.

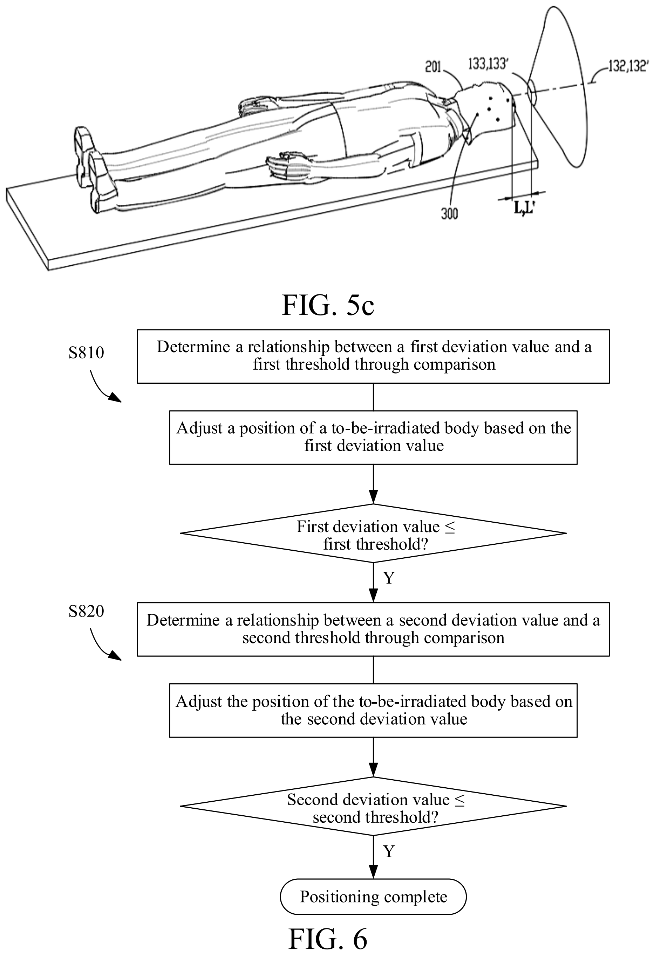

After the marking or before the marking, preferably, with reference to FIG. 5c, the to-be-irradiated body 200 is fixed at a corresponding position by using a position limiting member 201. Further, the to-be-irradiated body 200 is fixed to the movable positioning module 14 by using the position limiting member 201. The position limiting member 201 includes an inelastic fixing piece conforming to a shape of an affected part M of the to-be-irradiated body. Preferably, the inelastic fixing piece is a mouldable film, for example, a thermoplastic film, and may further have a visible part. The visible part is disposed near the neologism, to facilitate an operation such as setting a mark on the to-be-irradiated body 200. The position limiting member 201 may further include another component for fixing the to-be-irradiated body 200. The marker 300 may be directly disposed on the to-be-irradiated body 200, or the marker 300 is disposed on the position limiting member. For example, the marker 300 is disposed at a corresponding position on a surface of the thermoplastic film. In another embodiment, the marker 300 may be a marking feature for identifying the position limiting member 201, such as a visible point selected from prominent points of an outer contour of the position limiting member. In another embodiment, the position limiting member 201 may alternatively be a fixing and limiting member commonly used in photon, electronic radiation, proton, or heavy ion therapy.

Using a neoplastic area of the affected part M to be treated with radiation therapy as a center, preferably, markers 300 are dispersed in space around the affected part M and are attached to a visible surface of the to-be-irradiated body or the position limiting member 201. Further, a linear distance between the marker 300 and an edge of the neoplastic area does not exceed 500 mm, and in particular, does not exceed 200 mm. Further, a linear distance between centers of two adjacent markers 300 should be greater than a diameter of the marker 300, so that adjacent markers 300 do not overlap. The linear distance between centers of two adjacent markers 300 may exceed 5 mm, and in particular, may exceed 10 mm. It should be understood that the diameter of the marker 300 and the foregoing distance value may alternatively be smaller in another embodiment related to of small neologism therapy.

In a setting manner of this embodiment, there are at least three markers 300. When more than three markers 300 are selected, at least one marker 300 of the markers 300 and remaining markers 300 are not in a same plane. Further, if the affected part M of the to-be-irradiated body 200 is considered as a cuboid or another polyhedron, when most of a plurality of markers 300 are on one surface of the polyhedron, at least one marker is on a different surface. Further, for example, if the to-be-irradiated body is a patient, and when a neoplastic area to be treated with radiation therapy is located at the head of the to-be-irradiated body 200, namely, the patient, the markers 300 are disposed near the head of the patient 200. When at least one marker of the plurality of markers 300 is disposed on one side of the head, at least one marker is disposed on an opposite side of the head. Preferably, at least one marker is disposed on a front surface or another surface of the head. In another setting manner of this embodiment, if an area of the affected part M of the to-be-irradiated body 200 is offset to one side, when the markers 300 are disposed, it should be noted that any three markers should not be disposed on a same straight line as much as possible. In another setting manner of this embodiment, preferably, there may alternatively be at least four or more markers 300, the fourth marker or a subsequent marker is used for improving positioning accuracy in a special situation, and a position having good visibility and being more dispersed around the affected part M is selected as much as possible. For example, during scanning, the to-be-irradiated body 200 may be blocked by the reference object or another object such as the positioning module. When the markers 300 are easily partially blocked under a particular viewing angle, disposing the fourth marker or more markers facilitates visual alignment of positioning, and facilitates obtaining comprehensive scanned image data, to subsequently more precisely and quickly perform alignment and matching on image data of a positioning position of the patient.

S200: Obtain first image data of the to-be-irradiated body 200 and the marker 300.

Step S200 includes scanning the to-be-irradiated body 200 and the marker 300 by using the image module 50, to obtain the first image data including the to-be-irradiated body 200 and the marker 300. Step S200 may be performed by the first image module. The image module 50 may be a scanning instrument, a photographing instrument, or the like. The first image data is inputted into a treatment planning module 20, to calculate or match a corresponding treatment plan of the to-be-irradiated body, where the treatment plan includes information such as irradiation time, a preset dose, and a target position.

Further, the radiation therapy system 100 is integrally disposed in a medical facility. The medical facility includes a therapy room 101, and may further include a preparation room (not shown). The preparation room may be used as a collective name of all facilities for performing steps before determining that positioning is completed before the therapy, and may include a preparation room for obtaining the image, a preparation room for positioning, a medicine supply preparation room, and the like. It may be understood that in another embodiment, the preparation room may be disposed outside the medical facility, or may be disposed inside the medical facility. Step S200 is performed in the preparation room, and the image module 50 is disposed in the preparation room. It may be understood that the preparation room and the therapy room may be disposed in a same building of the medical facility, or may be disposed in different buildings. To ensure accuracy of positioning and determining of the treatment plan, a setting manner having a relative position between core devices basically the same as that in the therapy room 101 is preferably used for some facilities in the preparation room. The therapy room 101 includes a partition wall and a corresponding beam shaper or collimator. Correspondingly, the preparation room used for simulating positioning may include a simulated collimator with the same shape. To more accurate position the to-be-irradiated body 200 when the to-be-irradiated body is switched between different preparation rooms, or to more accurate position the to-be-irradiated body 200 when the to-be-irradiated body is switched between the preparation room and the therapy room, the preparation room or the therapy room may further be provided with a physical reference system for pre-positioning before obtaining the image, positioning, or irradiation, for example, the physical reference system may be a laser reference system. Reference points of physical reference systems disposed in the preparation room and the therapy room should be consistent to the maximum extent. For example, when laser reference systems are used in different spaces, three vertical laser planes formed by the laser reference systems have a unique intersection, and the intersection, as the reference point, should be calibrated to the same positions in the preparation room and the therapy room as much as possible.

In step S200, the first image data is obtained by using the scanning instrument in the preparation room, where the first image data includes at least image data of the to-be-irradiated body and the marker. In this embodiment, the image module 50 is a computed tomography (CT) device or a mobile scanning device. Further, the first image data further includes a first image three-dimensional model obtained by re-establishing image data (the first image data) obtained by using the CT. It may be understood that, a person skilled in the art may further use other medical image data. Provided that the medical image data can be re-established into a three-dimensional voxel prosthetic tissue model, the medical image data can be used in the radiation therapy system and the positioning method thereof disclosed in this embodiment, and in particular, can be used in the neutron capture therapy system and the positioning verification and correction method thereof. For a detailed process of establishing the three-dimensional voxel prosthetic tissue model based on the medical image data, refer to Patent Application Publication Number CN106474634A, entitled "MEDICAL IMAGE DATA-BASED GEOMETRIC MODEL ESTABLISHING METHOD" disclosed on March 8, 2017, which is incorporated herein by reference in its entirety. Further, step S200 further includes the following steps:
S201: Input the first image data including at least the to-be-irradiated body and the marker into the treatment planning module 20. The image module 50 directly or indirectly transmits the first image data to the treatment planning module 20.
S202: The treatment planning module 20 designs or optimizes the treatment plan based on the first image data. The treatment plan in step S202 includes, but is not limited to, the foregoing irradiation dose, irradiation time, and beam parameter. It may be understood that a treatment plan template including all or some of therapy parameters may be preset in the treatment planning module 20, and the treatment planning module 20 adjusts and optimizes the treatment planning parameters based on the first image data, or the treatment planning module 20 may calculate some or all of the therapy parameters based on the first image data. After the treatment planning module 20 determines the treatment plan, the user may also manually adjust or optimize the treatment plan.
S300: Determine a target position of the to-be-irradiated body based on the treatment plan obtained by using the treatment planning module 20.

Further, in step S200, the first image data has been obtained by using the image module 50. When the image module 50 is a CT device, a sliced image inside the to-be-irradiated body 200 can be obtained, and the treatment planning module 20 may improve or match a corresponding treatment plan based on the image data. Further, the first image data is re-established to obtain the first image three-dimensional model, and the treatment planning module 20 performs calculation or matching based on the first image data to obtain a planned target position of the first image three-dimensional model relative to a virtual reference object preset in the treatment plan. The target position includes position parameters (including a space parameter, a three-dimensional coordinate, and the like), model data, or the like used for positioning. For example, the position parameter may be converted into a motion coordinate, a motion path, a motion angle, and the like of a mechanical arm motion. The target position is an ideal preset therapy position of the to-be-irradiated body in the selected treatment plan.

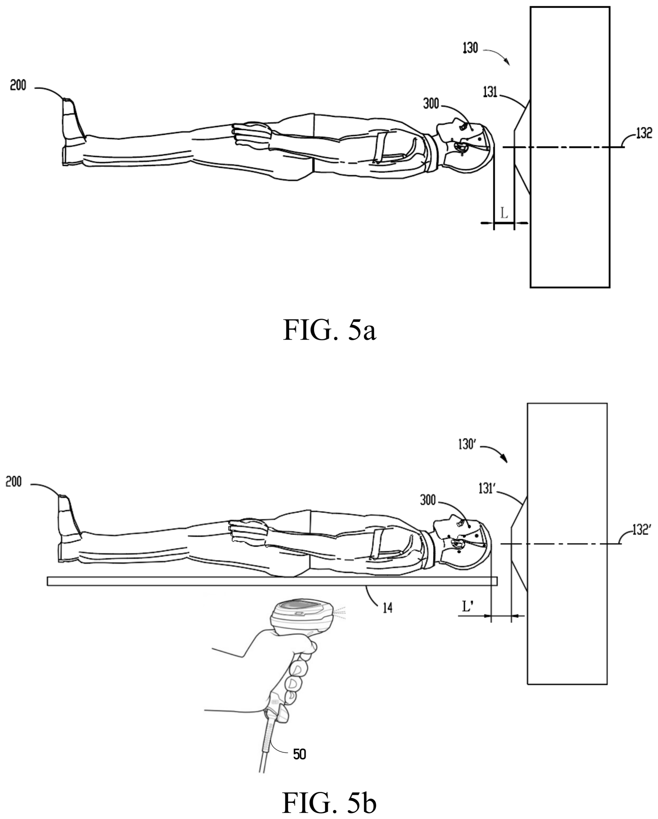

Specifically, step S300 further includes the following steps:
S301: The treatment planning module 20 performs calculation or matching based on the first image data to obtain the target position of the to-be-irradiated body 200.
S302: The image module 50 re-establishes the first image data to obtain the first image three-dimensional model.
S303: Adjust the virtual reference object model and the first image three-dimensional model to the target location. The target position described in this method includes a relative position parameter between the virtual reference object model and the first image three-dimensional model of the, and details are described in step S600. The treatment planning module 20 performs calculation based on various preset or selected therapy parameters, to obtain the target position of the to-be-irradiated body 200. For example, the therapy parameters include preset position coordinates of the virtual reference object model, an appropriate irradiation distance (such as a planned source-surface distance), and the first image three-dimensional model included in the first image data obtained through scanning. A fixed object existing in the therapy room 101 and/or the preparation room (not shown) or an object that can present same relative positions in two spaces should be selected as the virtual reference object model. For example, in this embodiment, a physical structure of an exit of the beam shaper or an exit of the collimator is selected, and three-dimensional modeling is performed on the exit structure of the beam shaper or the exit structure of the collimator to form the virtual reference object model, and the virtual reference object model and the first image three-dimensional model are placed at virtual target locations based on the relative position parameter in the treatment plan, as shown in FIG. 5a. It can be easily understood that in a BNCT device, the beam exit 13 is usually fixedly disposed in the therapy room, and a stable beam emitting center thereof can effectively ensure accuracy of therapy irradiation. Therefore, the beam exit 13 usually cannot be adjusted, and correspondingly, a simulated beam exit 131 exactly the same as the beam exit 13 is also fixedly disposed in the preparation room.

In another embodiment, another structure existing in the therapy room may alternatively be selected as the reference object. Correspondingly, a corresponding structure having the same relative position is disposed in the preparation room for reference, and the virtual reference object model 130 in the treatment planning module is also modeled correspondingly. When the to-be-irradiated body 200, the marker 300, and the reference object are scanned in step S200, the target position in step S300 may also be implemented by pre-positioning before the scanning.

S400: Move the to-be-irradiated body 200 to a positioning position according to the target position.

In step S400, an appropriate target position usually needs to be calculated or matched according to the treatment plan, and the position of the to-be-irradiated body 200 needs to be adjusted, so that the to-be-irradiated body 200 is substantial located at the preset therapy position, and subsequently, positioning verification is performed. The foregoing operation is simulated positioning. In the simulated positioning, the foregoing physical reference system may be used to switch between different spaces for reference, and then the positioning module 14 is used to perform position movement of the to-be-irradiated body in the simulated positioning process. The simulated positioning may be performed in the preparation room. A physical structure of an exit of a simulated beam shaper or a exit of a simulated collimator may be selected as a reference object 130' in the preparation room. After the simulated positioning is completed, the to-be-irradiated body 200 located in the preparation room usually needs to be moved to the therapy room 101 during formal therapy. It should be understood that to reduce deviation in the moving process, the simulated positioning may alternatively be directly performed in the therapy room 101. In this case, other space does not need to be disposed in the medical facility. In a therapy process or the simulated positioning process, the positioning position is adjusted based on the target position preset in the treatment plan, and an actual positioning position is obtained after the to-be-irradiated body 200 is moved into position. The positioning position may or may not coincide with the target position. When the positioning position coincides with the target position, it is in an optimal state, that is, the positioning position obtained in therapy or simulation has no deviation from the target position in the treatment plan. In this way, after positioning verification is completed, subsequent radiation therapy can be directly performed. However, generally, there is a deviation in the moving process. In other words, when the positioning position does not coincide with the target position, whether the positioning verification is completed directly determines whether subsequent irradiation therapy is safe and reliable. In this application, verification, correction, and adjustment are performed in the following step S800, to meet a corresponding requirement by using a plurality of adjustments. Therefore, the positioning position is a position at which therapy or simulation can be performed, and may include a plurality of positions, such as a pre-positioning position, a simulated positioning position, and a verification positioning position, at which therapy is assumed to be performed or actually can be performed. For example, the positioning position includes a first positioning position before step S500 is performed, and the positioning position further includes several intermediate positioning positions that are continuously adjusted. This is further described in steps S500 to S700.

S500: Obtain second image data of the to-be-irradiated body 200 and the reference object 130'.

As shown in FIG. 5b, in this embodiment, after the to-be-irradiated body 200 is moved to the positioning position for simulation or therapy according to the target position in the treatment plan, the to-be-irradiated body 200, the marker 300, and the reference object 130' are scanned by the image module 50, to obtain the second image data including the to-be-irradiated body 200, the marker 300, and the reference object 130'. Step S500 may be performed by the second image module. The second image module obtains image information of surfaces of the to-be-irradiated body 200, the reference object 130', and the marker 200 by using light reflection information. It should be noted that an object of a same type as the virtual reference object model 130 is selected as the reference object 130' for reference. In this embodiment, in step S300, the simulated beam exit 131 or the beam exit 131' is selected as the reference object 130', and corresponding modeling is performed in the treatment planning module, to obtain the virtual reference object model 130. The beam exit 131' may be disposed in the preparation room, or may be disposed in the therapy room 101. In another embodiment, a collimator or the like may be set as the reference object 130'.

Further, the second image data and the first image data are obtained in different manners. The image module 50 preferably uses a movable handheld three-dimensional scanner, to reestablish the second image data to obtain a second image three-dimensional model. The second image three-dimensional model includes an image three-dimensional model re-established based on the to-be-irradiated body 200, the marker 300, and the reference object 130'. Different from a manner of obtaining the first image data by using perspective scanning, in a scanning process, it is ensured as much as possible that the three-dimensional scanner moves to scan at a same speed, and scanning is completed when it is determined that no image is missing. A plurality of surface images obtained through scanning constitute three-dimensional information of the to-be-irradiated body 200, the marker 300, and the reference object 130'. A handheld three-dimensional scanner has a small structure, has high flexibility, and is convenient to use. The handheld three-dimensional scanner can scan, in a single use, a plurality of images of same positions on the to-be-irradiated body 200, the marker 300, and the reference object 130' at different angles, and establish three-dimensional information of the three including a position relationship, to directly obtain three-dimensional image data of the three, and does not need to be fixedly disposed in the preparation room or the therapy room, thereby effectively reducing spaces and device installation steps. In addition, image modules can also be shared in different spaces, and a movable operation manner further avoids a disadvantageous situation of damage caused by ray radiation received by devices and instruments in the therapy process. In another embodiment, a fixed scanner or an automatic scanner may alternatively be used. It may be understood that, provided that the second image data can be re-established into the three-dimensional image model, the scanner can be used in the radiation therapy system and the positioning method thereof disclosed in this application. This is not limited herein.

In an embodiment, the second image module includes at least one sensor and at least one light source, the light source is configured to at least emit light in a first spectral range, the first spectral range is near infrared light, and the second image module obtains image information on surfaces of the to-be-irradiated body, the reference object, and the marker by using information reflected from the light source to the sensor, and forms the second image data. Preferably, the second image module is a handheld scanning device, including at least two sensors and at least two light sources. A spectral range of at least one light source is non-visible light, and a spectral range of the other light source is visible light.

In another embodiment, the first image module obtains image information of the to-be-irradiated body, the reference object, and the marker by using a transmissive electromagnetic wave signal, to form the first image data.

S600: Determine first position information based on the treatment plan, and determine second position information based on the second image data.

The first position information includes at least first relative position information between the to-be-irradiated body 200 and the virtual reference object model 130, and first marker position information describing a position of the marker 300 in the preset target position. The second position information includes at least second relative position information between the to-be-irradiated body 200 and the reference object 130', and second marker position information describing a position of the marker 300 after the to-be-irradiated body actually reaches an actual positioning position through adjustment according to the preset target position. The first position information and the second position information may be respectively determined by the treatment planning module 20 and/or the processing module 40 based on the first image data and the second image data. The virtual reference object model may be preset in the treatment planning module 20 and/or the processing module 40.

Referring to FIG. 5a, the virtual reference object model 130 and the to-be-irradiated body 200 are spaced apart based on a preset parameter in the treatment plan, and the virtual reference object model 130 is disposed near a neoplastic area. In this embodiment, a neoplastic area of a human body as the to-be-irradiated body 200 is located at the head, and the virtual reference object model 130 is disposed near the top of the head, or may be disposed at a side of the head in another embodiment. Although FIG. 5c is a schematic diagram of a positioning position, for determining the target position in the treatment plan, reference may alternatively be made to schematic diagrams as shown in FIG. 5a and FIG. 5c, where the virtual beam exit 131 has a central point 133 passing through the exit, the central point 133 is located on an exit central line 132 (that is, a virtual beam transmission path) perpendicular to a plane in which the simulated beam exit 131 is located, and a linear distance from the exit central point 133 of the virtual beam exit 131 to the to-be-irradiated body 200 along the exit central line 132 is usually defined as a first source-surface distance L. The first relative position information includes at least the first source-surface distance L, and according to step S300, the first source-surface distance L is included in the treatment plan. Similarly, as shown in FIG. 5b and FIG. 5c, the reference object 130' and the to-be-irradiated body 200 are spaced apart based on the target position, and the reference object 130' is disposed near the neoplastic area. The beam exit 131' has a central point 133' passing through the exit, the central point 133' is located on an exit central line 132' (that is, a beam transmission path) perpendicular to a plane in which the beam exit 131' is located, and a linear distance from the exit central point 133' of the beam exit 131' to the to-be-irradiated body 200 along the exit central line 132' is usually defined as a second source-surface distance L'. The second relative position information includes at least the second source-surface distance L'. It should be noted that the preparation room or the therapy room 101 are not distinguished in FIG. 5b and FIG. 5c, and only positions of related structures are shown.

The first marker position information includes coordinate information of the marker 300 in the first image three-dimensional model or a relative position relationship between the marker and another reference object such as the virtual reference object model. The second marker position information includes coordinate information of the marker 300 in the second image three-dimensional model or a relative position relationship between the marker and another reference object such as the reference object.

S700: Calculate, based on the first position information and the second position information, a deviation value between a positioning position in actual therapy or in a simulation and the target position preset in the treatment plan.

The deviation value may include a first deviation value and a second deviation value, and may include deviation values of positions and/or angles. The processing module 40 calculates the first deviation value based on the first relative position information and the second relative position information, and the processing module 40 calculates the second deviation value based on the first marker position information and the second marker position information. In this embodiment, preferably, the first deviation value may be defined as position and angle differences between the second source-surface distance L' and the first source-surface distance L; the second deviation value may be defined as a set of position and angle deviation values of all markers 300 whose positioning positions in actual therapy or in the simulation are adjusted according to the target position in the treatment plan and all markers 300 corresponding to the target position preset in the treatment plan; and further, the second deviation value is a set of deviation values of position information of all markers 300 in the second image three-dimensional model and position information of all markers 300 corresponding to the first image three-dimensional model. It should be noted that, because the positioning position includes at least a positioning position for actual therapy, and may also include a plurality of positioning positions such as a positioning position for simulation obtained through adjustment according to the target position in the therapy process, the first deviation value and the second deviation value may be respectively a set of a plurality of deviation values.

S800: Adjust the position of the to-be-irradiated body based on the deviation value calculated by using the foregoing method.

S800 is a positioning correction method of a radiation therapy system after positioning verification based on image data is completed according to an embodiment of the present invention. Thresholds in the method are usually stored in the processing module 40, and the thresholds may include a first threshold and a second threshold. When the first deviation value is less than or equal to the first threshold, and the second deviation value is less than or equal to the second threshold, positioning is completed; and when the first deviation value is greater than the first threshold, and/or the second deviation value is greater than the second threshold, the position of the to-be-irradiated body is adjusted based on the first deviation value and/or the second deviation value, until the first deviation value is less than or equal to the first threshold and the second deviation value is less than or equal to the second threshold, positioning is completed. Preferably, as shown in FIG. 6, S800 further includes the following method:
S810: The processing module 40 determines a relationship between the first deviation value and the first threshold through comparison. When the first deviation value is greater than the first threshold, the position of the to-be-irradiated body 200 is adjusted based on the first deviation value, until the first deviation value is less than or equal to the first threshold. If the first deviation value is less than or equal to the first threshold, perform S820; otherwise, repeat the foregoing method. Further, a difference between the second source-surface distance L' and the first source-surface distance L may be set as the first deviation value, and a proper deviation value for the source-surface distance is set as a first threshold. Comparing the first deviation value with the first threshold, when the first deviation value is greater than the first threshold, a cause needs to be found and correction needs to be performed, and attempts need to be made to adjust the positioning position to enable the to-be-irradiated body to be as close to the target position in the treatment plan as possible, so that the first deviation value is less than or equal to the first threshold. If the position of the to-be-irradiated body 200 still cannot satisfy the foregoing requirement after being adjusted for a plurality of times, rematching or verification on the treatment plan needs to be considered, for example, whether setting of the threshold range is proper and whether the irradiation solution needs to be adjusted need to be considered, and comparison is performed again based on a modified treatment plan, until the first deviation value is less than the first threshold.
S820: The processing module 40 determines a relationship between the second deviation value and the second threshold through comparison. When the first deviation value is less than or equal to the first threshold, if the second deviation value is less than or equal to the second threshold, the positioning verification is completed, and subsequent irradiation therapy can be performed; if the second deviation value is greater than the second threshold, the position of the to-be-irradiated body 200 may be further adjusted based on a verification result of the second deviation value (for example, exceeding a range of the second threshold), to perform positioning correction on the to-be-irradiated body. By means of positioning adjustment for a plurality of times, the second deviation value is less than or equal to the second threshold, the positioning verification and correction are completed, and subsequent irradiation therapy can be performed. Preferably, in the verification step, the comparison in step S820 may be performed when it is ensured that the first deviation value is less than or equal to the first threshold in step S810. Further, deviation values are respectively calculated between the position information of all markers 300 in the second image three-dimensional model and the position information of all markers 300 corresponding to the first image three-dimensional model, each of second deviation values of the positions of all the markers 300 is compared with the second threshold, and the position of the to-be-irradiated body 200 is adjusted based on the the deviation values of all the markers 300 in sequence. Specifically, in the processing module 40, the second deviation value is calculated based on position and angle information between the position information of the markers 300 in the first image three-dimensional model and the position information of the corresponding markers 300 in the second image three-dimensional model. Accuracy of the first position information is more important for accuracy of the actual irradiation process. In a preferred manner of this embodiment, in a correction step, adjustment may be first performed based on the verification result of the first deviation value in step S810, step S820 is performed based on the position after the adjustment in step S810, and adjustment is performed based on the verification result of the second deviation value. In another embodiment, the relationship between the second deviation value and the second threshold may be verified first, or relationships between the first deviation value, the second deviation value, and corresponding thresholds may be verified at the same time. A sequence of S810 and S820 is not limited.

Further, before comparing the second deviation value with the second threshold, step S820 further includes the following method:
S821: Register the image three-dimensional model obtained by reestablishing the first image data and the second image data, so that models of the virtual reference object model 130 and the reference object 130' obtained through reestablishment coincide with each other. Specifically, the virtual reference object model 130 in the treatment plan is registered with an image model of the reference object 130' in the second image three-dimensional model, so that the two coincide with each other.
S822: Verify a coincidence degree of the first image data and the second image data.

When the second deviation value is less than the second threshold in step S820, the positioning is completed. It may be considered that, when the second deviation value is less than or equal to the second threshold in step S820, it means that the positioning position basically coincides with the target position, or a deviation degree of the positioning position is within an acceptable range for the therapy.

Further, when the deviation value of the markers 300 calculated in step S820 is large, for example, when the second deviation value is greater than a third threshold (where the third threshold is greater than the second threshold), step S800 further includes the following method:
S830: Register again the image three-dimensional model obtained by reestablishing the first image data and the second image data, so that models of the virtual reference object model 130 and the reference object 130' obtained through reestablishment coincide with each other. Specifically, the virtual reference object model 130 in the treatment plan is registered with an image model of the reference object 130' in the second image three-dimensional model, so that the two coincide with each other. S840: Establish a first three-dimensional parent coordinate system by using a central point of the reference object model as an origin; and establish a second three-dimensional parent coordinate system having a dimension the same as that of the first three-dimensional parent coordinate system by using a central point of the virtual reference object model 130 as an origin. In other words, reference origin and direction dimensions selected for the first three-dimensional parent coordinate system and the second three-dimensional parent coordinate system are the same, that is, the first three-dimensional parent coordinate system and the second three-dimensional parent coordinate system are established in completely the same manner. Because in step S830, the virtual reference object model 130 and the image model of the reference object 130' already coincide with each other, the virtual reference object model 130 and the reference object 130' are equivalent to a same reference object. The virtual reference object model 130 is used as an example for description below. Further, the simulated beam exit 131 or the therapy beam exit 131 may be selected as the virtual reference object model 130, and is correspondingly modeled in the treatment planning module. The central point 133 of the virtual reference object model 130 is aligned with an exit central point 133' of the simulated beam exit 131 or the therapy beam exit 131. The three-dimensional parent coordinate systems can be respectively established by using the exit center point 133 or 133' as origins, thereby respectively establishing X-Y-Z axes with completely the same dimensional directions.
S850: Separately establish a first three-dimensional child coordinate system of the first image data based on information about the marker 300 in the first image data, and establish, based on information about the marker 300 in the second image data, a second three-dimensional child coordinate system having an origin and a dimension the same as those of the first three-dimensional child coordinate system. The first three-dimensional child coordinate system and the second three-dimensional child coordinate system are established in the same manner, that is, completely the same X-Y-Z axes are established by using the same origin (for example, a same marker or feature point in two pieces of image data is selected as the origin) and directions as reference.
S860: When the first three-dimensional parent coordinate system and the second three-dimensional parent coordinate system are completely registered, separately integrate the first three-dimensional child coordinate system with the first three-dimensional parent coordinate system, and integrate the second three-dimensional child coordinate system with the second three-dimensional parent coordinate system.
S870: Calculate a deviation value of the first three-dimensional child coordinate system and the second three-dimensional child coordinate system in the two three-dimensional parent coordinate systems that are completely the same, and adjust the position of the to-be-irradiated body based on the deviation values. In this method, based on the first three-dimensional child coordinate system and the second three-dimensional child coordinate system, the deviation value of the actual positioning position of the to-be-irradiated body 200 relative to the target position in the treatment plan can be calculated. The deviation value may include data information of position and angle deviations. An adjustment value required in the correction step can be obtained based on the deviation value, so that correction and adjustment can be performed according to the adjustment value. Further, after the child coordinate systems are established, in addition to comparing the position information of the marker 300 in the two pieces of image data, coordinate axes of the first three-dimensional child coordinate system and the second three-dimensional child coordinate system may further be directly compared for comparison and verification.

Adjusting the position in step S800 may be that the positioning module 14 automatically adjusts the position of the to-be-irradiated body 200, or may be that the user adjusts the position based on feedback of the processing module 40. Further, the control module 30 may output a corresponding adjustment signal to the positioning module 14 based on a signal fed back by the processing module 40, and the positioning module 14 corrects the position of the to-be-irradiated body 200 based on position or angle deviation information included in the adjustment signal. The user may alternatively participate in the foregoing automatic adjustment process, or actively perform operational adjustment through feedback of the processing module 40.

The positioning method in this embodiment further includes a positioning verification and correction method of a radiation therapy system, and the positioning verification and correction method includes at least S700 and S800 in the foregoing method. In addition, in another embodiment, a sequence of the operation methods in FIG. 4 may not be limited.

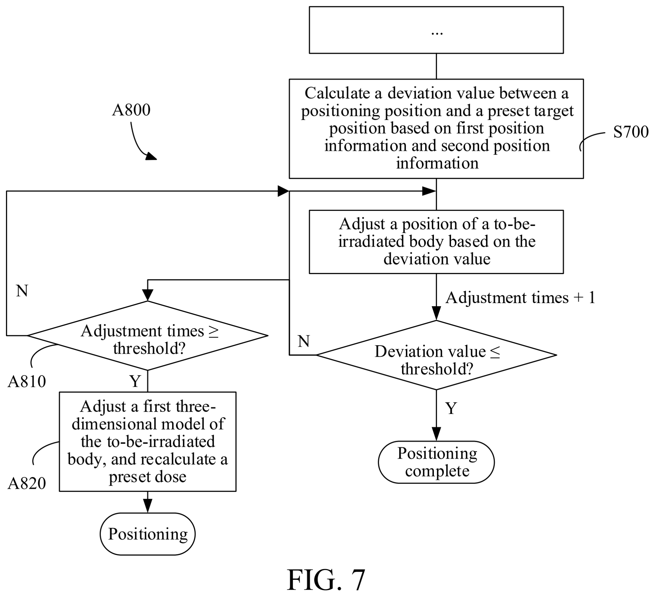

FIG. 7 is a schematic flowchart of a radiation therapy positioning method based on a three-dimensional scanning image according to another embodiment of this application. In this embodiment, most steps are basically the same as steps S100 to S700, and only steps different from those in the previous embodiment are described in detail. In comparison with the previous embodiment, S800 is replaced with the following step A800, or S800 may include step A800:
A800: Adjust the position of the to-be-irradiated body based on the deviation value, and if the deviation value is always greater than the preset threshold, that is, it is difficult to position the to-be-irradiated body according to the target position, the first image data of the to-be-irradiated body needs to be adjusted, and the treatment plan needs to be adjusted, to recalculate the preset dose. It should be noted that the to-be-irradiated body 200 needs to receive the preset dose at an actual positioning position as close to the target position as possible. As shown in FIG. 6, A800 specifically includes the following steps:
A810: Adjust the position of the to-be-irradiated body based on the deviation value, and each time the position is adjusted, real-time comparison of the deviation value is performed. In the previous embodiment, it has been described that the deviation value includes the first deviation value and the second deviation value. Preferably, position adjustment and determining based on the first deviation value are preferentially performed. In other words, when the first deviation value is less than or equal to the first threshold, comparison between the second deviation value and the second threshold and subsequent correction operations are performed. Otherwise, the treatment plan needs to be adjusted. In another embodiment, the first deviation value and the second deviation value may be comprehensively adjusted, or adjustment based on the second deviation value may be first performed. This is not limited herein. Further, a number of adjustment times is counted, and after the position of the to-be-irradiated body 200 is adjusted, the number of adjustment times increases by one.
A820: When the deviation value is greater than the threshold, determine whether the number of adjustment times is greater than or equal to the threshold, and if the number of adjustment times is less than the threshold, continue to perform step A810; or if the number of adjustment times is greater than or equal to the threshold, perform the following step A830. In another embodiment, if the number of adjustment times is less than or equal to the threshold, continue to perform step A810; or if the number of adjustment times is greater than the threshold, perform the following step A830.
A830: Adjust the first image data of the to-be-irradiated body 200, and recalculate, by the treatment planning module 20, the preset dose based on the first image data, where the current positioning position is a simulated or actual positioning position during final therapy. The first image three-dimensional model in the treatment plan obtained by re-establishing the first image data is adjusted based on the current actual positioning position and deviation value information, so that the first image three-dimensional model is in a position the same as the current positioning position. The treatment planning module 20 determines whether a deviation value of the radiation distance meets a requirement, and if the deviation value meets the requirement, recalculates a corresponding preset dose based on the position; or if the deviation value does not meet the requirement, a treatment plan needs to be re-adjusted or developed. In another embodiment, a determining standard of whether meeting the requirement is not limited to the deviation value of the irradiation distance, and whether a corresponding preset dose needs to be recalculated or a treatment plan needs to be re-adjusted or developed may alternatively be determined based on another index.

It should be understood that, although the steps are displayed sequentially according to the instructions of the arrows in the flowcharts of the foregoing embodiments, these steps are not necessarily performed sequentially according to the sequence instructed by the arrows. Unless explicitly described in the specification, execution of the steps is not strictly limited, and the steps may be performed in other sequences. In addition, at least a part of the steps in the flowcharts of the foregoing embodiments may include a plurality of steps or a plurality of stages. These steps or stages are not necessarily performed and completed at the same time, and may be performed at different times. Besides, these operations or stages may be not necessarily performed sequentially, and may be performed in turn or alternately with other steps or at least a part of operations or stages in other steps.

A person of ordinary skill in the art should understand that all or a part of the processes of the method in the foregoing embodiments may be implemented by a computer program instructing relevant hardware. The computer program may be stored in a non-volatile computer-readable storage medium. When the computer program is executed, the processes of the method in the foregoing embodiments are performed. Any reference to a storage module, a database, or another medium used in the various embodiments provided in this application may include at least one of a non-volatile memory or a volatile memory. The non-volatile memory may include a read-only memory (ROM), a magnetic tape, a floppy disk, a flash memory, an optical memory, a high-density embedded non-volatile memory, a resistive random access memory (ReRAM), a magnetoresistive random access memory (MRAM), a ferroelectric random access memory (FRAM), a phase change memory (PCM), a graphene memory, or the like. The volatile memory may include a random access memory (RAM), an external cache, or the like. For illustration rather than limitation, the RAM may be in various forms, for example, may be a static random access memory (SRAM) or a dynamic random access memory (DRAM). The database involved in the embodiments provided in this application may include at least one of a relational database and a non-relational database. The non-relational database may include a blockchain-based distributed database or the like, but is not limited thereto. The processor involved in the embodiments provided in this application may be a general-purpose processor, a central processing unit, a graphics processor, a digital signal processor, a programmable logic device, a quantum computing-based data processing logic device, or the like, but is not limited thereto.

The technical features in the foregoing embodiments may be combined in different manners to form other embodiments. For concise description, not all possible combinations of the technical features in the embodiment are described. However, the combinations of the technical features are all to be considered as falling within the scope described in this specification provided that they do not conflict with each other.

The foregoing embodiments only describe several implementations of this application, and are described in detail, but they are not to be construed as a limitation to the patent scope of this application. A person of ordinary skill in the art may further make variations and improvements without departing from the ideas of this application, which shall fall within the protection scope of this application. Therefore, the protection scope of the patent of this application shall be subject to the appended claims.

## Claims

1. A radiation therapy system, comprising:
an irradiation module, configured to: generate a therapy beam, and irradiate the therapy beam to a to-be-irradiated body;
an image module, configured to obtain image data of the to-be-irradiated body;
a treatment planning module, configured to generate a treatment plan based on the image data;
a positioning module, configured to move the to-be-irradiated body to a positioning position according to a target position obtained by using the treatment plan, wherein the image data comprises at least second image data of the to-be-irradiated body and a reference object at the positioning position; and
a processing module, configured to: determine first position information based on the treatment plan, determine second position information based on the second image data, and calculate a deviation value between the positioning position and the target position based on the first position information and the second position information.

2. The radiation therapy system according to claim 1, comprising a control module, configured to control the positioning module to adjust a position of the to-be-irradiated body based on the deviation value, wherein when the control module adjusts the position of the to-be-irradiated body based on the deviation value, if the deviation value is less than or equal to a preset threshold or the deviation value can be adjusted to be less than or equal to a preset threshold, a positioning verification succeeds; and if the deviation value is always greater than the threshold, the positioning verification fails, and the treatment plan needs to be re-adjusted.

3. The radiation therapy system according to claim 2, comprising a marker, wherein the marker is disposed or selected on the to-be-irradiated body, there are at least three markers, and any three of the markers are not on a straight line; and the image module comprises a first image module, wherein the first image module is configured to obtain first image data of the to-be-irradiated body and the marker, and the first image data is used for determining first position information in coordination with the treatment plan.

4. The radiation therapy system according to claim 3, wherein the image module further comprises a second image module, the second image module is different from the first image module, the second image module comprises at least one sensor and at least one light source, the light source is configured to at least emit light in a first spectral range, the first spectral range is near infrared light, and the second image module obtains image information on surfaces of the to-be-irradiated body, the reference object, and the marker by using information reflected from the light source to the sensor, and forms the second image data.

5. The radiation therapy system according to claim 4, wherein the treatment planning module comprises a preset virtual reference object model, the treatment plan determines the target position based on the first image data and the virtual reference object model, the first position information comprises first relative position information between the to-be-irradiated body and the virtual reference object model, and first marker position information describing the marker; and the second position information comprises second relative position information between the to-be-irradiated body and the reference object, and second marker position information describing the marker.

6. The radiation therapy system according to claim 5, wherein the deviation value comprises a first deviation value and a second deviation value, the processing module obtains the first deviation value based on the first relative position information and the second relative position information, the processing module obtains the second deviation value based on the first marker position information and the second marker position information, and the control module controls the positioning module to adjust the position of the to-be-irradiated body based on the first deviation value and/or the second deviation value.

7. A positioning method of a radiation therapy system, comprising:
determining a target position of a to-be-irradiated body based on a treatment plan;
moving the to-be-irradiated body to a positioning position according to the target position;
obtaining second image data of the to-be-irradiated body and a reference object;
determining first position information based on the treatment plan, and determining second position information based on the second image data;
calculating a deviation value between the positioning position and the target position based on the first position information and the second position information; and
adjusting a position of the to-be-irradiated body based on the deviation value.

8. The positioning method of a radiation therapy system according to claim 7, wherein the adjusting a position of the to-be-irradiated body based on the deviation value comprises: when adjusting the position of the to-be-irradiated body based on the deviation value, if the deviation value is less than or equal to a preset threshold or the deviation value can be adjusted to be less than or equal to a preset threshold, a positioning verification succeeds; and if the deviation value is always greater than the threshold, the positioning verification fails, and the treatment plan needs to be re-adjusted.

9. The positioning method of a radiation therapy system according to claim 7, wherein the radiation therapy system comprises a first image module, and the determining a target position of a to-be-irradiated body based on a treatment plan comprises: disposing or selecting a marker on the to-be-irradiated body; and obtaining first image data of the to-be-irradiated body and the marker by using the first image module; and the treatment plan comprises a preset virtual reference object model, and the treatment plan determines the target position based on the first image data and the virtual reference object model.

10. The positioning method of a radiation therapy system according to claim 9, wherein the first position information comprises first relative position information between the to-be-irradiated body and the virtual reference object model, and first marker position information describing the marker; and the second position information comprises second relative position information between the to-be-irradiated body and the reference object, and second marker position information describing the marker.

11. The positioning method of a radiation therapy system according to claim 10, wherein the deviation value comprises a first deviation value and a second deviation value; the first deviation value is obtained through calculation based on the first relative position information and the second relative position information, and the second deviation value is obtained through calculation based on the first marker position information and the second marker position information.

12. The positioning method of a radiation therapy system according to claim 11, wherein the threshold comprises a first threshold and a second threshold, and the adjusting a position of the to-be-irradiated body based on the deviation value comprises: when the first deviation value is less than or equal to the first threshold, and the second deviation value is less than or equal to the second threshold, positioning verification is completed; and when the first deviation value is greater than the first threshold, and/or the second deviation value is greater than the second threshold, the control module controls, based on the first deviation value and/or the second deviation value, the positioning module to adjust the position of the to-be-irradiated body, until the first deviation value is less than or equal to the first threshold and the second deviation value is less than or equal to the second threshold.

13. The positioning method of a radiation therapy system according to claim 11, wherein the threshold comprises a first threshold and a second threshold, and the adjusting a position of the to-be-irradiated body based on the deviation value comprises: when the first deviation value is greater than the first threshold, the control module controls, based on the first deviation value, the positioning module to adjust the position of the to-be-irradiated body, until the first deviation value is less than or equal to the first threshold; and when the second deviation value is greater than the second threshold, the control module controls, based on the second deviation value, the positioning module to adjust the position of the to-be-irradiated body, until the second deviation value is less than or equal to the second threshold.

14. The positioning method of a radiation therapy system according to claim 11, wherein the threshold comprises a first threshold and a second threshold, and the adjusting a position of the to-be-irradiated body based on the deviation value comprises: when the first deviation value is less than or equal to the first threshold, and the second deviation value is greater than the second threshold, the control module controls, based on the second deviation value, the positioning module to adjust the position of the to-be-irradiated body, until the second deviation value is less than or equal to the second threshold.

15. The positioning method of a radiation therapy system according to claim 12, wherein a first three-dimensional parent coordinate system is established by using a central point of a reference object model as an origin; a second three-dimensional parent coordinate system having a dimension the same as that of the first three-dimensional parent coordinate system is established by using a central point of the virtual reference object model as an origin; a first three-dimensional child coordinate system is established based on information about the marker in the first image data, and a second three-dimensional child coordinate system having an origin and a dimension the same as those of the first three-dimensional child coordinate system is established based on information about the marker in the second image data; when the first three-dimensional parent coordinate system and the second three-dimensional parent coordinate system are completely registered, the first three-dimensional child coordinate system is integrated with the first three-dimensional parent coordinate system, and the second three-dimensional child coordinate system is integrated with the second three-dimensional parent coordinate system; and a deviation value between the first three-dimensional child coordinate system and the second three-dimensional child coordinate system is calculated, and the position of the to-be-irradiated body is verified and adjusted based on the deviation value.
